# EUROPEAN PATENT APPLICATION

(11) **EP 4 847 601 A1**
(43) Date of publication of application: **02.12.2026**
(21) Application number: 25179448.3
(22) Date of filing: 28.05.2025
(51) Int. Cl.: C12N 1/20, A23K 10/18, A61K 35/744

(54) **NOVEL LACTIC ACID BACTERIA WITH THE ABILITY TO STRONGLY REDUCE THE PH OF THE SURROUNDING MEDIUM**

(71) Applicant: Evonik Operations GmbH, 45128 Essen (DE)
(72) Inventor: EHRING, Ellen, 48308 Senden (DE); TOM DIECK, Helke, 61381 Friedrichsdorf (DE); BRAUNER, Aileen Silvana, 33790 Halle/Westfalen (DE); VOGEL, Marie-Luise, 33659 Bielefeld (DE); SPECKMANN, Bodo, 32760 Detmold (DE); NIKOLOUDAKI, Olga, 39100 Bolzano (IT)
(74) Representative: Evonik Patent Association

(57) **Abstract**

The present invention concerns novel lactic acid bacteria with the ability to strongly reduce the pH of the surrounding medium and/or with the ability to produce high amounts of beneficial short chain fatty acids, in particular lactic acid, and preparations of such bacteria as well as compositions and products containing the bacteria or preparations thereof and further the use of the bacteria and preparations thereof in food, feed, pharmaceutical and cosmetical compositions as well as in personal care products, in particular for improving vaginal and/or female health.

## Description

The present invention concerns novel lactic acid bacteria with the ability to strongly reduce the pH of the surrounding medium and/or with the ability to produce high amounts of beneficial short chain fatty acids, in particular lactic acid, and preparations of such bacteria as well as compositions and products containing the bacteria or preparations thereof and further the use of the bacteria and preparations thereof in food, feed, pharmaceutical and cosmetical compositions as well as in personal care products, in particular for improving vaginal and/or female health.

The vaginal microbiome (VM) is a sophisticated and forceful microecosystem that constantly undergoes variations during the female menstrual cycle and woman's entire life. *Lactobacillus* is the dominant genus of a healthy VM, which produces diverse bacteriostatic compounds, lowers the vaginal pH due to production of lactic acid and competes with other urogenital microbial species (Witkin and Linhares 2017; Tachedjian et al. 2017). One classification of the VM supports the existence of five community state types (CST) (Ravel et al. 2011; Moosa et al. 2020), four of which are dominated respectively by *Lactobacillus crispatus* (CST-I), *Lactobacillus iners* (CST-III), *Lactobacillus gasseri* (CST-II) or *Lactobacillus johnsonii* (CST-V), while CST-IV is composed of a polymicrobial mixture of strict and facultative anaerobes not belonging to the *Lactobacillus* genus (Gardnerella, Atopobium, Prevotella, etc). Among other *Lactobacillus-related* species, *Levilactobacillus brevis* and *Limosilactobacillus fermentum,* but especially nomadic species like *Lactiplantibacillus plantarum* and *Lacticaseibacillus casei* or *Pediococcus species* can also be found in the vaginal microecosystem.

Bacterial vaginosis (BV) is characterized by a dysbiotic environment resulting from the shift from predominant lactobacilli to BV-related bacteria, such as *Gardnerella vaginalis* (Jones 2019). Most notable symptoms of BV are whitish grey discharges, fishy odor, increase of vaginal pH > 6-7, itchiness and burning (Bagnall and Rizzolo 2017). However, BV is also associated with more serious complications like preterm labor, miscarriage and endometriosis. The prevalence of BV is about 30% world-wide and partially unnoticed which is alerting (Javed, Parvaiz, and Manzoor 2019). Apart from bacterial infections, yeasts of *Candida* species that naturally inhabit the gastrointestinal and genitourinary tracts, upon certain conditions can colonize the vulvovaginal mucosa, giving rise to symptomatic vulvovaginal candidiasis (VVC), reducing quality of life for reproductive women, rising disbalances of mental health and sexual activity (Rodriguez-Cerdeira et al. 2019).

The use of *Lactobacillus-containing* probiotics to restore commensal VM has been proposed for the treatment and prophylaxis of bacterial urogenital infections and VVC (Shenoy and Gottlieb 2019). Administration of probiotic mixes for VVC has shown conflicting results depending on multiple factors such as the strains comprised in the mix, administration mode, patient's infection stage (e.g., acute, non-acute) and frequency of administration (Shenoy and Gottlieb 2019). Regarding BV, lactobacilli administered orally or intra-vaginally or in form of gel have been shown to have a preventive mode of action, whilst further investigation is necessary to reach definitive conclusions as to whether probiotics represent an effective and safe method for treatment. Nevertheless, lactobacilli-probiotics can play a crucial role in sustaining the vaginal pH low (3.5 - 4.5), compete with anaerobic pathogenic bacteria for nutrients, but also eliminating them with the secretion of antimicrobial substances such as bacteriocins, biosurfactants, lactic acid and H₂O₂ (Javed, Parvaiz, and Manzoor 2019).

Object of the present invention was to provide new microorganisms which are able to strongly reduce the pH of the surrounding environment, preferably better than microorganisms as disclosed in the state of the art, and/or to provide new microorganisms which are able to produce high amounts of beneficial acids, in particular a high amount of lactic acid, preferably a high amount of D-lactic acid, wherein the microorganisms are preferably able to produce higher amounts of such beneficial acids, in particular of lactic acid, preferably of D-lactic acid, than microorganisms as disclosed in the state of the art.

The object of the invention could be achieved by the microorganisms as described in the following. The microorganisms have originally been isolated from the female vagina. Due to their activity, the microorganisms have the potential to increase female health by production of high amounts of beneficial acids, in particular D-lactic acid. Production of the acids in particular lowers the pH at the site of application, which leads to deregulation of the growth of pathogenic bacteria.

Most of the lactic acid bacteria found in the vaginal microbiome produce L-lactate. Only a few strains are able to produce D-lactate. D-lactate produced by the vaginal microbiome is very beneficial, as it suppresses the vaginal extracellular matrix metalloproteinase inducer, adding a further level of protection against upper genital tract infections (Witkin and Linhares 2017).

Thus, a first subject of the present invention is a microorganism with the ability to strongly reduce the pH of the surrounding medium and a preparation thereof selected from the following group:
a) *Pediococcus pentosaceus* DSM 35171;
b) *Lactobacillus crispatus* DSM 35177;
c) *Lactiplantibacillus gasseri* DSM 35178;
d) *Lactiplantibacillus plantarum* DSM 35176;
e) a mutant of a strain according to (a) to (d) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
f) a preparation of a microorganism according to (a) to (e), wherein the preparation is preferably an optionally concentrated or dried fermentation broth, supernatant of a fermentation broth, ferment, cell lysate or cell extract of a microorganism according to (a) to (e), wherein the preparation may be cell-free or may contain living or dead cells or cell debris of such a microorganism or any combination thereof.

The microorganisms and microbial preparations according to the invention enhance human and in particular female health by their ability to strongly reduce the pH of the surrounding medium at the site of application. In addition they are preferably able produce a high amount of beneficial acids, in particular a high amount of lactic acid, preferably a high amount of D-lactic acid, wherein the acids can be made available to the body either by formation of such substances by the microorganisms *in vivo* at the site of application or upon administration of such substances, after they have been produced by the microorganisms of the invention *in vitro.*

The ability of the microorganisms of the invention to lower the pH of the surrounding medium is preferably as follows: If cultivated in an appropriate fermentation medium, in particular in MRS medium, starting with a cell density of 10⁹ cells per ml, the microorganisms of the invention are preferably able to lower the pH of the medium by at least 2.8 pH units, more preferably by at least 3.0 pH units, in the course of 24 hours of cultivation. In particular, the microorganisms of the invention are preferably able, if grown 24 hours, starting with a cell density of 10⁹ cells per ml, preferably in MRS medium, to lower the pH of the medium from 6.2 to at least 3.4, more preferably from 6.2 to at least 3.2. The mutants have preferably at least 90 % of the pH lowering capacity of the parent strains, from which they are derived, in particular if cultivated in MRS medium, starting from a cell density of 10⁹ cells per ml. More preferably, the mutants have at least the same pH lowering capacity than the parent strains, from which they are derived, in particular if cultivated in MRS medium, starting from a cell density of 10⁹ cells per ml.

The ability of the microorganisms of the invention to produce lactate is preferably as follows: If cultivated in an appropriate fermentation medium, in particular in MRS medium, to a cell density of 10⁹ cells per ml, the microorganisms of the invention are preferably able to produce lactic acid in an amount of at least 0.8 g/L, preferably in an amount of at least 1.0 g/L, more preferably in an amount of at least 1.2 g/L. In particular, the microorganisms of the invention are preferably able, if grown to a cell density of 10⁹ cells per ml, preferably in MRS medium, to produce D-lactic acid in an amount of at least 0.6 g/L, preferably in an amount of at least 0.8 g/L, more preferably in an amount of at least 1.0 g/L. The mutants have preferably at least 90 % of the lactic acid production capacity, in particular D-lactic acid production capacity, of the parent strains, from which they are derived, in particular if cultivated in MRS medium to a cell density of 10⁹ cells per ml. More preferably, the mutants have at least the same lactic acid production capacity, in particular D-lactic acid production capacity, than the parent strains, from which they are derived, in particular if cultivated in MRS medium to a cell density of 10⁹ cells per ml.

According to the invention the term "lactic acid" refers to lactic acid irrespective of the state of protonation. This means in particular that the depicted amounts of "lactic acid" refer to the total amount of lactic acid, including deprotonated lactate as present in the fermentation media. It is clear that the ratio of lactic acid to lactate as its deprotonated form depends on the pH of the fermentation medium.

The microorganisms and/or microbial preparations of the invention are preferably further characterized by at least one, in particular by at least two, three or four of the following further features:
- Ability to grow in presence of 0.15 wt.-% of bile;
- Ability to survive exposure to a pH of 2 for at least 2 hours, preferably with a survival rate of at least 5 %, more preferably of at least 10 %;
- Ability to survive the exposure to 3 g/L pepsin at 37°C for at least 2 hours, preferably with a survival rate of at least 5 %, more preferably of at least 10 %;
- Ability to survive the conditions of the gastrointestinal tract, preferably with a survival rate of at least 0.5 %, more preferably of at least 1 %;
- Ability to bind to cells of the vaginal tissue;
- Ability to inhibit the growth of pathogenic bacteria, in particular *Gardnerella vaginalis.*

In particular the strain *Pediococcus pentosaceus* DSM 35171 and mutants thereof are preferably further able to bind to cells of the vaginal tissue, in particular to VK2E6/E7 cells.

In particular the strains *Pediococcus pentosaceus* DSM 35171 and *Lactiplantibacillus plantarum* DSM 35176 and mutants of such strains are preferably further able to survive the conditions of the gastrointestinal tract. In particular, they are preferably able to survive exposure to a pH of 2 for at least 2 hours, preferably with a survival rate of at least 5 %, more preferably of at least 10 % and/or are preferably able to survive the exposure to 3 g/L pepsin at 37°C for at least 2 hours, preferably with a survival rate of at least 5 %, more preferably of at least 10 %.

In particular the strains *Lactiplantibacillus gasseri* DSM 35178 and *Lactiplantibacillus plantarum* DSM 35176 and mutants of such strains as well as preparations of such microorganisms are preferably further able to inhibit the growth of *Gardnerella vaginalis.*

The microorganisms and microbial preparations of the invention can be used in different ways, in particular in food and feed compositions, in particular in functional foods, pet foods, nutraceuticals and foods for special medical purposes (FSMP), in food and feed additives, in food and feed supplements, in pharmaceutical compositions, in cosmetical compositions, in particular for topical use, as well as in personal care products, which all are accordingly also subject matter of the invention. The functional food can in particular be a milk product like milk, cheese, yogurt or curd.

The application of the microorganisms and microbial preparations of the invention can accordingly occur in particular by oral application or topical application, wherein "topical application" in particular also comprises the application to the vaginal skin and/or mucosa.

Thus, a further subject of the invention is also a composition, in particular as mentioned before, containing a microorganism or microbial preparation according to the invention, in particular at least one microorganism or microbial preparation according to the invention, preferably selected from the following group:
a) *Pediococcus pentosaceus* DSM 35171;
b) *Lactobacillus crispatus* DSM 35177;
c) *Lactiplantibacillus gasseri* DSM 35178;
d) *Lactiplantibacillus plantarum* DSM 35176;
e) a mutant of a strain according to (a) to (d) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
f) a preparation of a microorganism according to (a) to (e), wherein the preparation is preferably an optionally concentrated or dried fermentation broth, supernatant of a fermentation broth, ferment, cell lysate or cell extract of a microorganism according to (a) to (e), wherein the preparation may be cell-free or may contain living or dead cells or cell debris of such a microorganism or any combination thereof.

Thus, a further subject of the invention is in particular also a pharmaceutical composition, containing a pharmaceutically acceptable carrier and a microorganism or microbial preparation according to the invention, in particular at least one microorganism or microbial preparation according to the invention, preferably selected from the following group:
a) *Pediococcus pentosaceus* DSM 35171;
b) *Lactobacillus crispatus* DSM 35177;
c) *Lactiplantibacillus gasseri* DSM 35178;
d) *Lactiplantibacillus plantarum* DSM 35176;
e) a mutant of a strain according to (a) to (d) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
f) a preparation of a microorganism according to (a) to (e), wherein the preparation is preferably an optionally concentrated or dried fermentation broth, supernatant of a fermentation broth, ferment, cell lysate or cell extract of a microorganism according to (a) to (e), wherein the preparation may be cell-free or may contain living or dead cells or cell debris of such a microorganism or any combination thereof.

A further subject of the invention is accordingly also a product, in particular a personal care product or a medical device, containing a microorganism or microbial preparation according to the invention, in particular at least one microorganism or microbial preparation according to the invention, preferably selected from the following group:
a) *Pediococcus pentosaceus* DSM 35171;
b) *Lactobacillus crispatus* DSM 35177;
c) *Lactiplantibacillus gasseri* DSM 35178;
d) *Lactiplantibacillus plantarum* DSM 35176;
e) a mutant of a strain according to (a) to (d) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
f) a preparation of a microorganism according to (a) to (e), wherein the preparation is preferably an optionally concentrated or dried fermentation broth, supernatant of a fermentation broth, ferment, cell lysate or cell extract of a microorganism according to (a) to (e), wherein the preparation may be cell-free or may contain living or dead cells or cell debris of such a microorganism or any combination thereof.

A further subject of the invention is therefore also a microorganism or microbial preparation of the invention, and a composition, in particular pharmaceutical composition, of the invention, in particular as mentioned before, for use in the treatment or prevention or mitigation of the course of a disease or condition selected from bacterial vaginosis, endometriosis, preterm labor, miscarriage, vaginal itchiness and burning, vulvovaginal candidiasis and vaginal infections, in particular vaginal infections by bacteria or yeasts of Candida species, mental, mood and/or sleep disorders or diseases, in particular pain, anxiety, stress and/or loss of motor control.

Due to the gut-vagina axis, the microorganisms and microbial preparations of the invention may in particular also have a positive impact on diseases or conditions of the gut, which are related to diseases or conditions of the vagina. Thus, a further subject of the invention is also a microorganism or microbial preparation of the invention, a composition, in particular pharmaceutical composition, of the invention, in particular as mentioned before, for use in the treatment or prevention or mitigation of the course of a disease selected from intestinal candidiasis, irritable bowel syndrome (IBS), intestinal infections, in particular intestinal infections by bacteria or yeasts of Candida species.

A further subject of the invention is therefore also the use of a microorganism or microbial preparation or of a composition of the invention in the manufacture of a pharmaceutical composition, in particular for treating or preventing or mitigating the course of a disease or condition selected from bacterial vaginosis, endometriosis, intestinal candidiasis, irritable bowel syndrome (IBS), preterm labor, miscarriage, vaginal itchiness and burning, vulvovaginal candidiasis and vaginal and intestinal infections, in particular vaginal and intestinal infections by bacteria or yeasts of Candida species, mental, mood and/or sleep disorders or diseases, in particular of pain, anxiety, stress and/or loss of motor control.

A further subject of the invention is therefore also the non-medical use of a microorganism or microbial preparation or of a composition or of a product of the invention for alleviating or preventing or mitigating the course of mental, mood and/or sleep disorders or disbalances, in particular anxiety, stress and/or loss of motor control and/or for supporting vaginal health, in particular for supporting the natural barrier function of the vaginal tissue, for protecting the vaginal tissue against specific pathogens, for promoting a healthy microbiome, for enhancing natural defenses, for preventing itching, burning and/or bad odor of the female genital area.

A further subject of the invention is accordingly also the use of a microorganism of the invention for producing lactic acid, in particular a mixture of D- and L-lactic acid, in particular by cultivating a microorganism of the invention in a suitable fermenter and preferably by using a suitable fermentation medium and wherein after the fermentation is ended the supernatant is preferably separated mechanically from the biomass to obtain a product containing the desired substances, wherein the supernatant may be concentrated and/or dried subsequently.

A further subject of the invention is accordingly also a method of producing a product, which contains lactic acid, in particular a mixture of L- and D-lactic acid, wherein a microorganism according to the invention is employed, wherein the microorganism is cultivated in a suitable medium and wherein after the fermentation is ended the supernatant is preferably separated mechanically from the biomass to obtain a product containing the desired substances, wherein the supernatant may be concentrated and/or dried subsequently.

The microorganisms of the invention were identified by screening of naturally occurring isolates and were deposited at the DSMZ (Leibniz-Institute DSMZ-German Collection of Microorganisms and Cell Cultures, Inhoffenstraße 7B, 38124 Braunschweig, Germany) under the provisions of the Budapest Treaty on the International Recognition of the Deposit of Microorganisms for the Purpose of Patent Procedure in the name of the applicant Evonik Operations GmbH on September 23, 2024. Originally, the microorganisms were isolated from samples of the female vagina or food sources.

The strain *Pediococcus pentosaceus* DSM 35171 exhibits the following characterizing sequences: a 16S rDNA sequence according to SEQ ID NO: 1, an rpoB sequence according to SEQ ID NO: 2, a gyrB sequence according to SEQ ID NO: 3, a groL sequence according to SEQ ID NO: 4, an ychF sequence according to SEQ ID NO: 5, a phoH sequence according to SEQ ID NO: 6 and a sequence according to SEQ ID NO: 7 which is located on the site G1416_G1777_07580 and codes for a hypothetical protein.

Accordingly, the mutants of the strain DSM 35171 preferably exhibit a 16S rDNA sequence, an rpoB sequence, a gyrB sequence, a groL sequence, an ychF sequence, a phoH sequence and/or said sequence located on the site G1416_G1777_07580 coding for a hypothetical protein, which are at least 99 or 99.5 %, more preferably at least 99.8 or 99.9 %, above all 100 % identical to the respective sequences of the strain DSM 35171 as mentioned above.

The mutants of the strain DSM 35171 have preferably further a sequence identity of at least 99 %, more preferably of at least 99.5 %, in particular of at least 99.8 or 99.9 % to the genomic DNA of the strain DSM 35171.

The strain *Lactobacillus crispatus* DSM 35177 exhibits the following characterizing sequences: a 16S rDNA sequence according to SEQ ID NO: 8, an rpoB sequence according to SEQ ID NO: 9, a gyrB sequence according to SEQ ID NO: 10, a groL sequence according to SEQ ID NO: 11, an ychF sequence according to SEQ ID NO: 12, a phoH sequence according to SEQ ID NO: 13, a sequence according to SEQ ID NO: 14 which is located on the site G1447_G1779_01615 and codes for a IS110 family transposase and a sequence according to SEQ ID NO: 15 which is located on the site G1447_G1779_05155 and codes for a glycosyltransferase family 2 protein.

Accordingly, the mutants of the strain DSM 35177 preferably exhibit a 16S rDNA sequence, an rpoB sequence, a gyrB sequence, a groL sequence, an ychF sequence, said sequence located on the site G1447_G1779_01615 coding for a IS110 family transposase and/or said sequence located on the site G1447_G1779_05155 coding for a glycosyltransferase family 2 protein, which are at least 99 or 99.5 %, more preferably at least 99.8 or 99.9 %, above all 100 % identical to the respective sequences of the strain DSM 35177 as mentioned above.

The mutants of the strain DSM 35177 have preferably further a sequence identity of at least 99 %, more preferably of at least 99.5 %, in particular of at least 99.8 or 99.9 % to the genomic DNA of the strain DSM 35177.

The strain *Lactiplantibacillus gasseri* DSM 35178 exhibits the following characterizing sequences: a 16S rDNA sequence according to SEQ ID NO: 16, an rpoB sequence according to SEQ ID NO: 17, a gyrB sequence according to SEQ ID NO: 18, a groL sequence according to SEQ ID NO: 19, an ychF sequence according to SEQ ID NO: 20, a phoH sequence according to SEQ ID NO: 21 and a sequence according to SEQ ID NO: 22 which is located on the site G1439_G1780_05200 and codes for a membrane protein.

Accordingly, the mutants of the strain DSM 35178 preferably exhibit a 16S rDNA sequence, an rpoB sequence, a gyrB sequence, a groL sequence, an ychF sequence, a phoH sequence and/or said sequence located on the site G1439_G1780_05200 coding for a membrane protein, which are at least 99 or 99.5 %, more preferably at least 99.8 or 99.9 %, above all 100 % identical to the respective sequences of the strain DSM 35178 as mentioned above.

The mutants of the strain DSM 35178 have preferably further a sequence identity of at least 99 %, more preferably of at least 99.5 %, in particular of at least 99.8 or 99.9 % to the genomic DNA of the strain DSM 35178.

The strain *Lactiplantibacillus plantarum* DSM 35176 exhibits the following characterizing sequences: a 16S rDNA sequence according to SEQ ID NO: 23, an rpoB sequence according to SEQ ID NO: 24, a gyrB sequence according to SEQ ID NO: 25, a groL sequence according to SEQ ID NO: 26, an ychF sequence according to SEQ ID NO: 27, a phoH sequence according to SEQ ID NO: 28 and a sequence according to SEQ ID NO: 29 which is located on the site G1598_03250 and codes for a DNA methyltransferase.

Accordingly, the mutants of the strain DSM 35176 preferably exhibit a 16S rDNA sequence, an rpoB sequence, a gyrB sequence, a groL sequence, an ychF sequence, a phoH sequence and/or said sequence located on the site G1598_03250 coding for a DNA methyltransferase, which are at least 99 or 99.5 %, more preferably at least 99.8 or 99.9 %, above all 100 % identical to the respective sequences of the strain DSM 35176 as mentioned above.

The mutants of the strain DSM 35176 have preferably further a sequence identity of at least 99 %, more preferably of at least 99.5 %, in particular of at least 99.8 or 99.9 % to the genomic DNA of the strain DSM 35176.

The mutants of the invention can principally be obtained by any kind of method, i.e., by GMO- or non-GMO methods, but preferably the mutants are not genetically modified, i.e., non-GMO. This means that the mutants of the deposited strains are preferably either also naturally occurring microorganisms or spontaneous mutants of such naturally occurring microorganisms, in particular of the deposited strains, or microorganisms which are obtained by another method which is classified as non-GMO. The term "spontaneous mutant" refers to mutants that arise from naturally occurring microorganisms and/or parent strains without genetically modifying the microorganisms by applying classical gene technological and/or biotechnological methods like site-directed mutagenesis. Such spontaneous mutants may be obtained by classical methods of natural selection, such as growing the microorganisms in the presence of UV light and/or by applying high temperature or protoplast formation and/or in the presence of a certain antibiotic to which the parent strain is susceptible. The generation of such mutants *in vitro* may in particular also occur by adaptive laboratory evolution ("ALE").

Spontaneous mutants might further, but less preferably, be obtained by using mutagens, i.e., chemical substances which induce the formation of mutants. As formation of spontaneous mutants by using mutagens is less preferred, in a preferred embodiment of the invention the spontaneous mutants and/or non-GMO mutants are obtained without the use of such mutagens. If the mutants are obtained by applying gene technological and/or biotechnological methods like site-directed mutagenesis, then preferably methods are applied which are classified as non-GMO. A non-GMO method according to the invention is preferably characterized in that the method does not involve introduction of heterologous genetic information into the microorganism. In a particularly preferred embodiment of the invention the microorganisms of the invention are naturally non-occurring mutants, in particular non-GMO and/or spontaneous mutants as defined before. The mutants of the invention have preferably the same characteristics like the parent strain from which they are derived.

The microorganisms of the invention are preferably present, in particular in the preparations and compositions of the invention, as living cells, i.e., in the vegetative state. But besides that the cells can also be used in non-living, inactivated form, or as a combination of living and inactivated cells, as also the inactivated cells are expected to still have a certain probiotic effect.

Accordingly, preparations of the microorganisms of the invention, which are a preferred subject of the invention, may be preparations containing intact cells, inactivated cells, cell debris or mixtures thereof. Further, the preparations may also be cell-free preparations, wherein the cell-free preparations may contain cell debris or may be free of cell debris. Particularly preferred examples for preparations of the microorganisms of the invention are the fermentation broth, as obtained after finishing the fermentation of the cells, the supernatant of the fermentation broth, which is obtained by separating all or the major part of the cells from the fermentation broth, as well as cell lysates and cell extracts, i.e. cytosol preparations, which can be obtained by breaking the microbial cells. Such preparations may also be used in concentrated or dried form, wherein the dried form has preferably a total dry matter content of at least 90 wt.-%, more preferably of at least 95 wt.-%.

The term "fermentation broth" according to the invention refers to the product of a cultivation of bacteria in a suitable fermentation medium. Methods for producing such fermentation broths are well known to those skilled in the art. The fermentation broths of the present invention can for example be obtained by culturing the strains by using the media, conditions and methods as described in US 6,060,051, EP 0287699, US 2014/0010792 or in the FAO Report 179 (2016): "Probiotics in Animal Nutrition". Conventional large-scale microbial culture processes include submerged fermentation, solid state fermentation, or liquid surface culture. Typically, fermentation is carried out, until a certain cell density is reached like an optical density (OD 600) of about 5 to 25, more preferably 10 to 22, above all 15 to 20. The fermentation broths preferably contain cells in an amount of 1x10³ to 1x10¹³ CFU, more preferably in an amount of 1x10⁵ to 1x10¹² CFU, above all in an amount of 1x10⁷ to 1x10¹¹ CFU per ml of fermentation broth.

The term "fermentation broth" according to the invention refers to the direct product of the cultivation, i.e. a suspension preferably containing cells in the amount as mentioned in the previous paragraph, as well as to concentrated and dried forms of such a fermentation broth, wherein the dried form ("dried fermentation broth") preferably has a total dry matter content of at least 95 wt.-%.

Drying of the preparations of the invention can be carried out by applying methods as generally known to those skilled in the art, in particular by evaporation of water, freeze-drying, lyophilization, spray drying, spray granulation, fluidized bed drying, vacuum drying, tray drying, drum drying and combinations thereof. After drying, the dried preparations as obtained can be further worked up, in particular by grinding and/or granulation.

Cell-free preparations of the microorganisms of the invention can be obtained by centrifugation, filtration, in particular ultrafiltration or microfiltration, decantation and/or flotation of the fermentation broth or of a suspension as obtained after breaking the microbial cells. Depending on the technique used, these cell-free preparations may not be completely devoid of cells, but may still comprise a smaller amount of cells, in particular up to 20 wt.-% or up to 10 wt.-% of cells. As the cells produce and secret compounds like metabolites, enzymes and/or peptides into the surrounding medium, the supernatants, extracts and lysates of the cells comprises a mixture of such compounds, in particular metabolites, enzymes and/or peptides, as secreted and/or produced by the cells.

Cell lysates can be prepared either directly by breaking the cells of an optionally dried fermentation broth or by breaking the cells after first separating the cells from the fermentation broth mechanically, in particular by filtration, centrifugation and/or decantation.

Breaking of the cells can be carried out by applying techniques as known to those of skill in the art, in particular mechanically, chemically and/or enzymatically. Depending on the degree of force applied, a composition comprising only ruptured cells or a composition comprising a mixture of cell debris and intact cells is obtained. Homogenization of the cells may be realized for example by applying high pressure, high pH, osmotic shock, heat shock, sonication, homogenization or shearing, in particular by utilizing means selected from French cell press, sonicator, homogenizer, microfluidizer, ball mill, rod mill, pebble mill, bead mill, high pressure grinding roll, vertical shaft impactor, industrial blender, high shear mixer, paddle mixer, and/or polytron homogenizer.

After breaking of the cells, the cell debris and remaining cells, if any, can optionally be separated from the cytosol preparation thus obtained to obtain a cell extract, i.e. a cytosol preparation, which is free of cells and cell debris.

The fermentation broths of the invention, from which the cells have been removed, i.e. the cell-free supernatant and metabolites containing fractions of the fermentation broth, are also denoted as "postbiotics", "metabiotics" or "biogenics".

A further specific preparation of the invention is a preparation containing inactivated cells, i.e. cells which are not able to grow anymore. Preparations containing inactivated cells are also known as "paraprobiotics". Such preparations can be obtained for example by heat and/or pH inactivation of the cells, preferably by heat and/or pH inactivation of a fermentation broth containing the cells.

Inactivation of the cells can be carried out, e.g., by heat treatment and/or by adjusting an acid or an alkaline pH. Heat inactivation of the cells is preferably carried out by increasing the temperature to between 60°C and 80°C and incubation for at least 30 minutes. pH inactivation at acidic pH is preferably carried out by lowering the pH to at least 3 by addition of an acid like 5M H₂SO₄ and incubation for at least 30 minutes, preferably at least one hour. pH inactivation at alkaline pH is preferably carried out by increasing the pH to at least 10 and incubation for at least 30 minutes, preferably at least one hour. Alternatively, inactivation of the cells may also be carried out by applying gamma- or UV-irradiation. In one specific embodiment of the invention a fermentation broth is used, in which at least 90 %, more preferably at least 95 or 99 %, in particular all bacterial cells are inactivated. Correspondingly, in this embodiment of the invention a fermentation broth is used which preferably contains no viable cells, at all. Such a product, which contains mainly or exclusively inactivated cells, is preferably used in cosmetical and/or topical applications and is called "ferment" in the context of the present invention.

The preparations preferably comprise a high amount of lactic acid, in particular a high amount of D-lactic acid. They preferably may also comprise further organic acids, in particular acetic acid. In addition they may comprise metabolites, enzymes and/or peptides as produced by the microorganisms of the invention.

In a preferred embodiment of the invention, the microbial preparations of the invention comprise DNA of the microbial strain(s), from which they were obtained, wherein they preferably comprise the complete DNA, in particular the complete genomic DNA, of said strain(s), so that they can be assigned to said strain(s).

Preferably according to the invention always an effective amount of the microorganisms, microbial preparations, in particular fermentation broths, compositions and/or products of the invention is used in the embodiments of the invention.

Compositions and products of the invention preferably contain the microorganisms of the invention in an amount of from 1x10² to 1x10¹² CFU, more preferably in an amount of 1x10³ to 1x10¹⁰ CFU, above all in an amount of 1x10⁴ to 1x10⁹ CFU and/or in an amount of from 0.1 to 20 wt.-%, preferably 0.2 to 15 wt.-%, in particular 0.5 to 10 wt.-%, above all 1 to 5 wt.-%.

In context of the present invention, the term "effective amount" preferably refers to an amount which effects at least one beneficial effect to the vaginal tissue and/or to female health, in comparison to the vaginal tissue and/or female health in case that the individuum has not been exposed to the microorganisms, microbial preparations, compositions and/or products of the invention, irrespective of the kind of application of the microorganisms, microbial preparations, compositions or products of the invention, but may have been exposed to the same treatment and/or the same compositions or products depleted by such microorganisms or microbial preparations.

As due to the gut-vagina axis, orally administered microorganisms can reach the vaginal region and vice versa, thus the oral application of the microorganisms may have a positive effect on vaginal health, while the vaginal application of the microorganisms may vice versa have a positive effect on gut health. Furthermore, as transmittal of the microorganisms does also occur by direct body contact, thus a positive effect may also be reached by administering the microorganisms to the partner of the person in need instead of administering the microorganisms directly to the person in need of the treatment.

In context with food and feed compositions of the invention, the term "effective amount" may also refer to an amount which effects at least one beneficial effect to the gastrointestinal tract of human beings or animals in comparison to the gastrointestinal tract of human beings or animals that have not been exposed to the microorganisms, microbial preparations and/or compositions of the invention, but may have been exposed to the same treatment and/or to the same food or feed compositions depleted by such microorganisms or microbial preparations.

In context with cosmetical compositions of the invention, the term "effective amount" may also refer to an amount which effects at least one beneficial effect to the human skin in comparison to a human skin that has not been exposed to the microorganisms, microbial preparations, compositions and/or products of the invention, but may have been exposed to the same treatment and/or to the same cosmetical composition depleted by microorganisms or microbial preparations.

The microorganisms and microbial preparations of the invention can also be provided in combination with a suitable carrier, wherein the carrier is preferably an inert formulation ingredient added to improve recovery, efficacy, or physical properties and/or to aid in packaging or administration of the microorganisms or microbial preparations. Such carriers may be used individually or in combination and can be added either as part of the fermentation medium, in the course of the fermentation or after the fermentation of the microorganisms has been ended.

The carrier is preferably selected from anti-caking agents, antioxidants, bulking agents, binders, structurants, coatings and/or protectants. Examples of useful carriers include polysaccharides (in particular starches, maltodextrins, celluloses, methylcelluloses, gums like guar gum, xanthan gum and gum arabic, wheat middlings, corn cob meal, chitosan and/or inulins), protein sources (in particular skim milk powder, sweet-whey powder, gelatine and/or soy flour), protein hydrolysates (in particular gelatine, yeast extract and/or peptones like soy peptone), peptides, sugars (in particular lactose, trehalose, sucrose, dextrose and/or maltose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), silicates (in particular clays, zeolites, Fuller's earth, clintpolite, montmorillonite, perlite, vermiculite, diatomaceous earth, talc, bentonites, kaolin clay, silica in particular precipitated silica, hydrophobic silica and/or hydrophilic silica, and/or silicate salts like aluminium, magnesium and/or calcium silicate), silica gel, silica dioxide, activated carbon, lignite, magnesium and calicum oxide.

Compositions and products of the invention, in particular as mentioned before, preferably contain at least one further additive which helps to improve vaginal and/or female health, wherein the at least one further additive is preferably selected from estriol, mannose, lactic acid, glycogen, inulin, xylo-oligosaccharid (XOS), zinc, selenium, vitamin A, vitamin B1 , vitamin B2, vitamin B6, vitamin B12, biotin, niacin, folic acid, pantothenate, vitamin C (ascorbic acid), vitamin D, cinnamon, cranberry extract, myrrh extract, chamomile extract, sour cherry extract, oat extract and yeast extract.

While estriol helps to fight vaginal atrophy, mannose and cranberry extract help to prevent or alleviate infections of the urinary tract. Lactic acid, ascorbic acid and glycogen stabilize the vaginal pH and have antioxidative activity. Zinc, vitamin D as well as extracts of myrrh and chamomile support the function or regneration of the vaginal mucosa.

The compositions and products of the invention may contain at least one further additive selected from carriers, in particular as mentioned before, further probiotics, further prebiotics, enzymes, further vitamins, further minerals, amino acids, peptides, further plant extracts and algal extracts.

The at least one further probiotic microorganism is preferably contained in the compositions of the invention, if present, in an amount of about 1x10² to about 1x10¹⁰ CFU/g, more preferably in an amount of about 1x10⁴ to about 1x10⁹ CFU/g, in particular in an amount of 1x10⁶ to 1x10⁸ CFU/g. It is used preferably in form of an optionally dried fermentation broth.

Such further probiotic microorganisms are preferably bacteria selected from the genera Bacillus, Lactobacillus, Lacticaseibacillus, Lactiplantibacillus, Levilactobacillus, Bifidobacterium, Enterococcus or Pediococcus. Probiotic microorganisms of the genus Bacillus are preferably selected from the species *Bacillus subtilis, Bacillus licheniformis, Bacillus paralicheniformis, Bacillus lentus, Bacillus pumilus, Bacillus laterosporus, Bacillus coagulans, Bacillus alevi, Bacillus cereus, Bacillus badius, Bacillus thurigiensis, Bacillus amyloliquefaciens, Bacillus velezensis, Bacillus nakamurai* and *Bacillus siamensis.* Probiotic bacteria of the genus Enterococcus are preferably of the species *Enterococcus faecium.* Probiotic bacteria of the genus Pediococcus are preferably of the species *Pediococcus acidilactici.* Further suitable probiotic microorganisms are *E*. *coli* Nissle and *Clostridium butyricum.*

The compositions of the invention preferably contain at least one further, preferably at least two further, probiotic microorganism(s) or preparation(s) thereof with beneficial activity on female health, in particular with respect to the vaginal skin or mucosa, wherein the at least one or two further microorganism(s) is/are selected from microorganisms with the ability to inhibit the growth of pathogens of the vagina, in particular the growth of *Gardnerella vaginalis* and/or *Candida albicans,* and/or from microorganisms which are able to effectively bind to cells of the vaginal tissue.

In a preferred embodiment of the invention, the composition contains at least one further microorganism or microbial preparation with the ability to inhibit the growth of *Candida albicans* and/or *Gardnerella vaginalis,* wherein the microorganism is preferably a lactic acid bacterium and wherein the microorganism or microbial preparation is more preferably selected from the following group:
a) *Limosilactobacillus fermentum* DSM 35179;
b) *Lactiplantibacillus plantarum* DSM 35180.
c) a mutant of a strain according to (a) or (b) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
d) a preparation of a microorganism according to (a) to (c), wherein the preparation is preferably an optionally concentrated or dried fermentation broth, supernatant of a fermentation broth, ferment, cell lysate or cell extract of a microorganism according to (a) to (c), wherein the preparation may be cell-free or may contain living or dead cells or cell debris of such a microorganism or any combination thereof.

The ability of the microorganisms as mentioned before to inhibit the growth of *Gardnerella vaginalis* is preferably as follows: If three volume units of G. *vaginalis* cells in ATCC medium 1685 at an OD620 of 0.25 are mixed with one volume unit of a cell-free supernatant of the microorganisms as mentioned before, which was obtained by culturing such microorganisms in MRS medium to an OD620 of 0.25 and subsequent neutralization (pH 7.0) and sterile-filtration of the fermentation broth, then the subsequent growth of *G*. *vaginalis* is preferably inhibited by at least 20 %, more preferably by at least 30 or 50 %, in particular by at least 70, 80 or 90 %, very preferably completely, in comparison to a negative control, preferably if measured 46 hours after the mixing, and in particular if tested in an assay as described in working example 5.

The ability of the microorganisms to inhibit the growth of *Candida albicans* is preferably as follows: If three volume units of *C*. *albicans* cells provided in Sabouraud medium at an OD600 of 0.1 are mixed with one volume unit of a cell-free supernatant of the microorganisms, which was obtained by culturing the microorganisms in MRS medium to an OD620 of 0.25 and subsequent sterile-filtration of the fermentation broth, then the subsequent growth of *C*. *albicans* is inhibited by at least 10 %, more preferably by at least 15 %, in comparison to a negative control, preferably if measured 46 hours after the mixing, and in particular if tested in an assay as described in working example 6.

The mutants have preferably at least 90 % of the inhibitory activity of the parent strain, from which they are derived, against *Candida albicans* and/or against *Gardnerella vaginalis,* in particular against *Candida albicans* and *Gardnerella vaginalis.* More preferably the mutants have at least the same inhibitory activity, in particular exactly the same inhibitory activity or even a better inhibitory activity, against *Candida albicans* and/or *Gardnerella vaginalis,* in particular against *Candida albicans* and *Gardnerella vaginalis,* than the parent strain, from which they are derived.

In a further preferred embodiment of the invention, the composition contains at least one microorganism or microbial preparation with the ability to bind to cells of the vaginal tissue, in particular to VK2E6/E7 cells, wherein the microorganism is preferably a lactic acid bacterium and wherein the microorganism or microbial preparation is preferably selected from the following group:
a) *Lacticaseibacillus rhamnosus* DSM 35175;
b) a mutant of a strain according to (a) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
c) a preparation of a microorganism according to (a) or (b), wherein the preparation is preferably an optionally concentrated or dried fermentation broth, supernatant of a fermentation broth, ferment, cell lysate or cell extract of a microorganism according to (a) or (b), wherein the preparation may be cell-free or may contain living or dead cells or cell debris of such a microorganism or any combination thereof.

The microorganisms as mentioned before, which are able to bind to cells of the vaginal tissue, are preferably able to bind to VK2E6/E7 cells, more preferably they are able to bind better to cells of the vaginal tissue, in particular to VK2E6/E7 cells, than the reference strain ATCC53103. Even more preferably they have a binding capacity with respect to VK2E6/E7 cells which is at least 20, 30 or 50 % better than the binding capacity of the reference strain ATCC53103, in particular when tested in an assay as described in working example 4.

Preferably, the microorganisms show a binding capacity of at least 10 %, more preferably of at least 12 or 14 % with respect to VK2E6/E7 cells, in particular when tested in an assay as disclosed in working example 4.

The ability of the microorganisms as mentioned before to bind to VK2E6/E7 cells is preferably as follows: If VK2E6/E7 cells provided in an amount of 2 x 10⁵ cells adhered to the surface of a well having a diameter of 32 mm² are incubated in said well at 37°C for 90 minutes with 5% CO2 with 500 µl Keratinocyte-Serum Free Medium added with 0.1 ng/ml Epidermal growth factor, 0.05 mg/ml bovine pituitary extract and 0.4 mM CaCl2, containing the microorganisms at a concentration of 5x10⁶ cells/ml, then the amount of microorganisms which show binding to VK2EG/E7 cells is preferably at least 10 %, more preferably at least 20 %, in particular if tested in an assay as disclosed in working example 4.

The mutants have preferably at least 90 % of the binding capacity of the parent strain, from which they are derived, to cells of the vaginal tissue, in particular to VK2E6/E7 cells. More preferably the mutants have at least the same binding capacity, in particular exactly the same binding capacity or even a better binding capacity, to cells of the vaginal tissue, in particular to VK2E6/E7 cells, than the parent strain, from which they are derived.

The strains DSM 35179, DSM 35180 and DSM 35175 were identified by screening of naturally occurring isolates and were deposited at the DSMZ (Leibniz-Institute DSMZ-German Collection of Microorganisms and Cell Cultures, Inhoffenstraße 7B, 38124 Braunschweig, Germany) under the provisions of the Budapest Treaty on the International Recognition of the Deposit of Microorganisms for the Purpose of Patent Procedure in the name of the applicant Evonik Operations GmbH on September 23, 2024. Originally, the microorganisms were isolated from samples of the female vagina or food sources.

The strain *Limosilactobacillus fermentum* DSM 35179 exhibits the following characterizing sequences: a 16S rDNA sequence according to SEQ ID NO: 30, an rpoB sequence according to SEQ ID NO: 31, a gyrB sequence according to SEQ ID NO: 32, a groL sequence according to SEQ ID NO: 33, an ychF sequence according to SEQ ID NO: 34, a phoH sequence according to SEQ ID NO: 35 and a sequence according to SEQ ID NO: 36, which is located on the site G1412_G1781_02670 and codes for a preprotein translocase subunit SecE.

Accordingly, the mutants of the strain DSM 35179 preferably exhibit a 16S rDNA sequence, an rpoB sequence, a gyrB sequence, a groL sequence, an ychF sequence, a phoH sequence and/or said sequence located on the site G1412_G1781_02670 coding for a preprotein translocase subunit SecE, which are at least 99 or 99.5 %, more preferably at least 99.8 or 99.9 %, above all 100 % identical to the respective sequences of the strain DSM 35179 as mentioned above.

The mutants of the strain DSM 35179 have preferably further a sequence identity of at least 99 %, more preferably of at least 99.5 %, in particular of at least 99.8 or 99.9 % to the genomic DNA of the strain DSM 35179.

The strain *Lactiplantibacillus plantarum* DSM 35180 exhibits the following characterizing sequences: a 16S rDNA sequence according to SEQ ID NO: 37, an rpoB sequence according to SEQ ID NO: 38, a gyrB sequence according to SEQ ID NO: 39, a groL sequence according to SEQ ID NO: 40, an ychF sequence according to SEQ ID NO: 41, a phoH sequence according to SEQ ID NO: 42 and a sequence according to SEQ ID NO: 43, which is located on the site G1856_10555 and codes for a DNA methylase.

Accordingly, the mutants of the strain DSM 35180 preferably exhibit a 16S rDNA sequence, an rpoB sequence, a gyrB sequence, a groL sequence, an ychF sequence, a phoH sequence and/or said sequence located on the site G1856_10555 coding for a DNA methylase, which are at least 99 or 99.5 %, more preferably at least 99.8 or 99.9 %, above all 100 % identical to the respective sequences of the strain DSM 35180 as mentioned above.

The mutants of the strain DSM 35180 have preferably further a sequence identity of at least 99 %, more preferably of at least 99.5 %, in particular of at least 99.8 or 99.9 % to the genomic DNA of the strain DSM 35180.

The strain *Lacticaseibacillus rhamnosus* DSM 35175 exhibits the following characterizing sequences: a 16S rDNA sequence according to SEQ ID NO: 44, an rpoB sequence according to SEQ ID NO: 45, a gyrB sequence according to SEQ ID NO: 46, a groL sequence according to SEQ ID NO: 47, an ychF sequence according to SEQ ID NO: 48, a phoH sequence according to SEQ ID NO: 49 and a sequence according to SEQ ID NO: 50 which is located on the site G1158_G1778_11060 and codes for a hypothetical protein.

Accordingly, the mutants of the strain DSM 35175 preferably exhibit a 16S rDNA sequence, an rpoB sequence, a gyrB sequence, a groL sequence, an ychF sequence, a phoH sequence and/or said sequence located on the site G1158_G1778_11060 coding for a hypothetical protein, which are at least 99 or 99.5 %, more preferably at least 99.8 or 99.9 %, above all 100 % identical to the respective sequences of the strain DSM 35175 as mentioned above.

The mutants of the strain DSM 35175 have preferably further a sequence identity of at least 99 %, more preferably of at least 99.5 %, in particular of at least 99.8 or 99.9 % to the genomic DNA of the strain DSM 35175.

Mutants and preparations of the strains DSM 35179, DSM 35180 and DSM 35175 are preferably obtained as described before for the strains DSM 35171, DSM 35177, DSM 35178 and DSM 35176.

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used according to the invention and which may aid in the digestion of feed, are preferably selected from phytases (EC 3.1 .3.8 or 3.1 .3.26), xylanases (EC 3.2.1 .8), galactanases (EC 3.2.1 .89), galactosidases, in particular alpha-galactosidases (EC 3.2.1.22) and beta-galactosidases, proteases (EC 3.4), lipases, phospholipases, in particular phospholipases A1 (EC 3.1 .1 .32), A2 (EC 3.1.1.4), C (EC 3.1.4.3), and D (EC 3.1.4.4), lysophospholipases (EC 3.1 .1.5), amylases, in particular alpha-amylases (EC 3.2.1.1 ), lysozymes (EC 3.2.1 .17), glucanases, in particular beta- glucanases (EC 3.2.1 .4 or EC 3.2.1 .6), glucoamylases, cellulases, pectinases, glutamate decarboxylases, or any mixture thereof.

Vitamins which may be used according to the invention are for example vitamin A, vitamin D3, vitamin E, vitamin K, e.g., vitamin K3, vitamin B12, biotin, choline, vitamin B1 , vitamin B2, vitamin B6, niacin, folic acid and pantothenate, e.g. Ca-D-pantothenate, or combinations thereof.

Minerals which may be used according to the invention are for example boron, cobalt, chloride, chromium, copper, fluoride, iodine, iron, manganese, molybdenum, selenium, zinc, calcium, phosphorous, magnesium, potassium, or sodium, or combinations thereof.

Amino acids which may be used according to the invention are in particular proteinogenic amino acids as known to those skilled in the art, in particular L-threonine, L-methionine, L-valine, L-lysine and L-tryptophan.

Dipeptides which may be used according to the invention are in particular any dipeptides of proteinogenic amino acids.

Plant extracts are according to the invention preferably selected from extracts from cranberry, myrrh, chamomile, sour cherry, oat, broccoli, olive fruit, pomegranate, blackcurrant, blueberry, bilberry, sea buckthorn, camu camu, boysenberry, curcuma, ginger, garlic, grape seeds, acai berry, aronia, goji berry, horseradish, rice, beans, kudzu, bamboo, hokkaido, boswellia serrata, spirulina, panax ginseng, cannabidiol, rose hip, pu erh, sea grass, sencha, echinacea and green tea leaves.

Compositions of the invention, in particular feed, food and pharmaceutical compositions of the invention, preferably comprise at least one further feed or food additive.

Suitable typical feed or food additives which may be contained in the feed, food and pharmaceutical compositions according to the invention include one or more of the following: proteins, carbohydrates, fats, feed concentrates, silage, mashfeed, probiotics, prebiotics, enzymes, vitamins, immune modulators, milk replacers, minerals, amino acids, carriers, in particular as mentioned above, coccidiostats, acid-based products and/or medicines, in particular antibiotics.

Carbohydrates containing components which may be used according to the invention are for example forage, roughage, wheat meal, corn meal, sunflower meal or soya meal, and mixtures thereof.

Proteins containing components which may be used according to the invention are for example soya protein, pea protein, wheat gluten, corn gluten, rice, canola meal, meal of marine animals, in particular fishmeal, meal of terrestrial animals, and mixtures thereof. "Meal of marine animals" includes meat meal, meat and bone meal, blood meal, liver meal, poultry meal, silkworm meal, silkworm pupae meal and combinations thereof.

Fats are typically provided as oils of marine animals, vegetable oils or oils of microorganisms, in particular oils of microalgae, or combinations thereof. Examples of vegetable oils are soybean oil, rapeseed oil, sunflower seed oil, canola oil, cottonseed oil, flaxseed oil and palm oil. Oils of marine animals include fish oil as well as oil of krill, bivalves, squids or shrimps and further fatty oils from fish of the families Engraulidae, Carangidae, Clupeidae, Osmeridae, Scombridae and/or Ammodytidae. Examples of oils of microalgae are in particular oil of Labyrinthulea, preferably oil of Schizochytria or Thraustochytria. Besides the isolated oils the defatted biomass itself may also be used as fat source, i.e. in particular the meal of a marine animals, preferably fishmeal, or a plant meal, in particular soybean meal, rapeseed meal, sunflower meal, canola meal, cottonseed meal and/or flax seed meal.

Proteins containing components which additionally contain fats which may be used according to the invention are for example fish meal, krill meal, bivalve meal, squid meal or shrimp shells, as well as combinations thereof.

Immune modulators which may be used are for example antibodies, cytokines, spray-dried plasma, interleukins, or interferons, or combinations thereof.

The compositions of the invention may further comprise betaine and/or choline and/or other physiologically effective methyl group donors. The compositions may further comprise polyunsaturated fatty acids, in particular DHA and/or EPA.

Thus, a further embodiment of the invention is also a method of preparing a feed, food or pharmaceutical composition comprising mixing a compound or composition of the invention, in particular in an amount effective to enhance animal health, in particular gut health, with feed or food ingredients, such as proteins, lipids and/or carbohydrates, and optionally further beneficial substances, preferably as mentioned before, to provide a feed, food or pharmaceutical product. This method may comprise also a pelleting and/or extruding step. Thus, in a preferred embodiment of the invention the feed compositions are feed or food pellets and/or feed or food extrudates.

The food composition of the invention can in particular be a functional food, a food for special medical purposes, a nutraceutical or a dietary supplement. The dietary supplement can in particular be a pill, a tablet, a capsule or a liquid.

The compositions of the invention, in particular the food and feed compositions, may also be contained in a capsule, in particular in a bile-resistant capsule.

The compositions of the invention, in particular the food and feed compositions, may also comprise a coating, in particular an enteric coating, wherein the enteric coating preferably comprises one or more of the following substances: methyl acrylate-methacrylic acid copolymers, cellulose acetate phthalate (CAP), cellulose acetate succinate, Hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate, zein.

A further subject of the invention is also the use of the microorganisms or microbial preparations or feed compositions of the invention for feeding animals, in particular selected from ruminants, swine, poultry and aquaculture animals.

A further subject of the invention is accordingly also a method of feeding animals, in particular selected from ruminants, swine, poultry and aquaculture animals, wherein microorganisms, microbial preparations or feed compositions of the invention are employed.

The cosmetical composition of the invention is preferably a topical skin care composition.

According to the invention the term "skin" refers preferably to the skin itself, particularly to the human skin, but may also refer to the mucosa and skin adnexa, in so far that they include living cells, particularly hair follicles, hair roots, hair bulbs, the ventral epithelial layer of the nail bed (lectulus) as well as sebaceous glands and perspiratory glands.

The cosmetic or pharmaceutical composition according to the invention can be in any form suitable for application on the skin, e.g., a soap, a lotion, a serum, a spray, a jelly, a cream, a gel, a paste, a pomade, a balm, an ointment, a foam, a mousse, an emulsion, a stick, a suppository, a patch, a powder, a cleaning fluid or cleaning milk, a deodorant, an anti-perspirant, a salve, a hair conditioner or a shampoo. The composition can also be applied in a mask or in a band-aid, particularly in a gel reservoir mask or band-aid or matrix mask or band-aid.

The cosmetical compositions of the invention preferably have a pH in the range of between 3 to 8, in particular in the range of 4.0 to 8.0, more preferably in the range of 4.5 to 7.4, particularly preferably in the range of 5.0 to 7.2. The pH of the composition is preferably determined at 22°C after stirring for five minutes using a pH electrode calibrated in accordance with ISO 4319 (1977).

The application area can be the skin of any part of the body, particularly the facial skin, the scalp, the skin on hands and feet, the skin under the arms, as well as the mucous membrane, wherein the skin and the mucosa in the vaginal area are particularly preferred.

In a very preferred embodiment of the invention the cosmetical composition is a composition for topical application to the vaginal mucosa and is in particular selected from suppositories, creams and gels.

The cosmetical composition of the invention is preferably an emulsion, in particular a W/O or an O/W emulsion. This means that the composition contains preferably at least one surface-active substance as emulsifier or as dispersion agent. Emulsifiers act at the interphase to produce water or oil-stable adsorption layers that protect the dispersed droplets against coalescence and thereby stabilize the emulsion. Thus, emulsifiers, like surfactants, are composed of hydrophobic and hydrophilic molecular moieties. Hydrophilic emulsifiers preferably form O/W emulsions and hydrophobic emulsifiers preferably form W/O emulsions. An emulsion is understood to mean a dispersion of a liquid in the form of droplets in another liquid using an energy input to afford interphases stabilized with surfactants. The choice of this emulsifying surfactant or emulsifier depends on the materials being dispersed and the respective external phase as well as the fineness of the emulsion.

In addition to or instead of the emulsifiers as mentioned above the cosmetical compositions according to the invention may also comprise foaming, non-ionic, zwitterionic, anionic and/or cationic surfactants and/or biosurfactants as further surface-active ingredients.

The cosmetical or pharmaceutical skin care composition of the invention preferably comprises at least one further ingredient selected from phospholipids, sphingoid bases, free fatty acids, protein hydrolyzates, polymers, light stabilizers, anti-perspirants, deodorants, peptides, amino acids, vitamins, extracts from plants and algae, cosmetic oils, emollients, antioxidants, preservatives, thickeners, viscosity regulators, stabiliziers, hydrotropes, solids, fillers, film formers, conditioners, insect repellents, self-tanning agents, odor absorbers, solvents, perfumes and dyes.

A further subject of the invention is accordingly also the use of the microorganisms, microbial preparations and/or cosmetical compositions of the invention for treating the human skin, in particular the skin and/or mucosa in the vaginal region.

A further subject of the invention is accordingly also a method of treating the human skin topically, in particular the skin and/or mucosa in the vaginal region, wherein the microorganisms, microbial preparations and/or compositions of the invention are employed.

As production of lactic acid, in particular D-lactic acid, and lowering the pH are important topics for female health, thus a further subject of the invention are also personal care articles which contain microorganisms or microbial preparations of the invention, wherein the personal care articles are preferably disposable absorbent articles, in particular selected from sanitary napkins, panty liners, tampons, diapers, incontinent pads, breast pads, perspiration pads, human or animal waste management devices and interlabial pads.

The personal care articles preferably contain a water-absorbing gelling material, wherein the water-absorbing gelling material is preferably a water-absorbing composition comprising water-absorbing polymer structures, wherein the water-absorbing polymer structures are preferably based on partly neutralized, crosslinked polyacrylates. Very preferably, the personal care articles contain superabsorbent polymers.

Superabsorbent polymers (superabsorbers) are water-insoluble crosslinked polymers which are capable of absorbing and retaining under pressure large amounts of aqueous liquids, in particular body fluids, preferably urine or blood, with swelling and formation of hydrogels. In general, these uptakes of liquid are an amount of water at least 10 times or even at least 100 times the dry weight of the superabsorber or superabsorbent compositions. Due to these characteristic properties, these polymers are very suitable to be used in sanitary articles. A comprehensive overview of superabsorbers and superabsorbent compositions, their use and their preparation is given by F. L. Buchholz and A. T. Graham (editors) in "Modern Superabsorbent Polymer Technology", Wiley-VCH, New York, 1998.

Suitable water-absorbing gelling materials and methods of preparing them are described or mentioned for example in WO 2009/040106.

The microorganisms and microbial preparations can further be immobilized on the water-absorbing gelling material with aid of a binder, in particular with aid of an organic or inorganic binder. Suitable binders for this purpose are also disclosed in WO 2009/040106 and are preferably selected from thermoplastic hot melt adhesives and hydrophilic, preferably water-soluble, polymers. Thermoplastic hot melt adhesives which are preferably employed are described more in detail WO 2005/011860. Hydrophilic, preferably water-soluble, polymers which can be employed are in particular polyvinyl alcohols, which can be obtained by partial or complete hydrolysis of polyvinyl acetate, or polyalkylene glycols, in particular polyethylene glycols having a molecular weight in a range of from 1,000 to 50,000 g/mol, particularly preferably in a range of from 1,500 to 25,000 g/mol and most preferably in a range of from 2,000 to 15,000 g/mol.

### Working examples

### Example 1: Determination of lactic acid production by Pediococcus pentosaceus DSM 35171, Lactobacillus crispatus DSM 35177, Lactiplantibacillus gasseri DSM 35178, Lactiplantibacillus plantarum DSM 35176

Vaginal swabs of 67 female donors were collected and plate count from the vaginal swabs was performed as soon as the samples arrived in the laboratory, using de Man, Rogosa and Sharpe (MRS) medium (MRS-OXOID, Italy; 10g/L peptone, 4 g/L yeast extract, 8 g/L meat extract, 20 g/L glucose, 2 g/L dipotassium phosphate, 5 g/L sodium acetate, 2 g/L ammonium acetate, 0.2 g/L magnesium sulfate, 0.05 g/L manganese sulfate, 1 g/L polysorbate 80; pH adjusted to pH 6.2) agar plates (ad 15 g/L agar-agar). MRS agar plates were supplemented with 0.05 % L-cysteine. The plates were incubated under aerobic and anaerobic conditions (37 °C, 48-72 h). The anaerobic conditions were created by using anaerobic jars supplemented with Anaerocult C (Merck, Milan, Italy). Strains were isolated and collected for further use.

DSM 35171, DSM 35177, DSM 35178 and DSM 35176 were cultivated in 10mL MRS medium for 24 h at 37 °C under anaerobic conditions. The anaerobically grown cells were cultivated in a 15 ml tube without agitation. For the determination of lactic acid production anaerobically grown cells were adjusted to 10⁹ cells/mL (OD₆₂₀ of 0.25). After the adjustments the cell-free supernatants (CFSs) were separated by centrifugation (7500 rpm, 10 min) and were fractionated in two 1 ml aliquots. One aliquot was neutralized to pH 7.0 (n-CFSs) with NaOH and sterilized by using a 0.22 µm filter. The other aliquot was preserved at the original acidic pH of ca. 4.0 (o-CFSs) and was sterilized by using a 0.22 µm filter. All aliquots of CFSs were stored at -20°C no more than one week.

The quantification of lactic acid in the o-CFSs was done using the L (+) and D (-) lactate dehydrogenase kit (Megazyme International Ireland Ltd., Co. Wicklow, Ireland), following the manufacturer's instructions, with some modifications. Briefly, 96-well plates were used to prepare the samples that were run in duplicate. Each well was filled with 150 µL of distilled water, 10 µL of sample, 50 µL of solution 1 (buffer), 10 µL of solution 2 (NAD+) and 2 µL of solution 3 (D-GPT). A blank was prepared using the same solutions, but putting 160 µL of water instead (no sample) and run in duplicate, as well. The plate was gently mixed (5 sec) and, after 3 min, the absorbance (A₁) was read using the Infinite nano+ spectrophotometer (Tecan, Austria). After, 2 µL of suspension 5 (D-LDH) were added both to the samples and the blank. The plate was mixed gently (5sec) and incubated for 5 min and the absorbance (A₂) was read again with the Infinite nano+ spectrophotometer (Tecan, Austria). Finally, 2 µL of suspension 4 (L-LDH) were added both to the samples and the blank. After a gentle mix (5 sec), the plate was incubated for 10 min and the absorbance (A₃) was read again with the Infinite nano+ spectrophotometer (Tecan, Austria). The concentration of D- and L-lactic acid were calculated determing the absorbance differences (A2-A1 and A3-A2, respectively), together with the final volumes (224 µL for D-lactic acid and 226 µL for L-lactic acid), molecular weight of lactic acid (90.1 g/mol), extinction coefficient of NADH at 340 nm = 6300 (mol⁻¹ cm⁻¹), light path (0.56 cm), and sample volume (0.1 µL).

**Table 1: D-, L- and Total Lacate. The quantification of lactic acid in the non-sterile o-CFSs was done using the L (+) and D (-) lactate dehydrogenase kit (Megazyme International Ireland Ltd., Co. Wicklow, Ireland). Mean values were given. O-CFS: original acidic pH of the cell-free supernatant of a 24h cultivation.**

| **Strain** | **Species** | **Average D-Lactate [g/L]** | **Average L-Lactate [g/L]** | **Total L-/D-Lactate [g/L]** |
|---|---|---|---|---|
| **DSM 35177** | *Lactobacillus crispatus* | 0,87 | 0,57 | 1,45 |
| **DSM 35178** | *Lactobacillus gasseri* | 0,69 | 0,61 | 1,30 |
| **DSM 35176** | *Lactiplantibacillus plantarum* | 1,40 | 0,19 | 1,58 |
| **DSM 35171** | *Pediococcus pentosaceus* | 0,15 | 0,68 | 0,83 |
| **Evonik strain 4** | *Lactobacillus crispatus* | 0,12 | 0,17 | 0,29 |
| **Evonik strain 5** | *Lactobacillus gasseri* | 0,35 | 0,35 | 0,69 |
| **Evonik strain 6** | *Lactiplantibacillus plantarum* | 0,62 | 0,66 | 1,28 |
| **Competitor strain 1** | *Lactobacillus crispatus* | 0,42 | 0,53 | 0,95 |
| **Competitor strain 2** | *Lactobacillus crispatus* | 0,54 | 0,50 | 1,04 |
| **Competitor strain 3** | *Lactobacillus gasseri* | 0,59 | 0,52 | 1,10 |

As can be seen, the strains DSM 35176, DSM 35177 and DSM 35178 have the ability to produce very high total amounts of L- and D-Lactate in the selected *in vitro* system. The production of lactate causes a reduction of the total pH and also deregulates pathogenic growth. Many lactic acid bacteria found in the vaginal microbiome produce L-lactate, but only very few are known to produce D-lactate. Production of D-lactate is very beneficial, as it suppresses the vaginal extracellular matrix metalloproteinase inducer, adding a further level of protection against upper genital tract infections

DSM 35176 is the highest D-lactate producer (1.4 g/L). Moreover the strain is producing L-lactate and shows the highest total lactate production of all tested strains (1.58 g/L). None of the other strains of the species *Lactiplantibacillus plantarum* shows such a high production. The production of lactate is very strain specific. Besides the strain DSM 35176, the strains DSM 35177 and DSM 35178 have comparable lactate production capacity (1.45 and 1.30 g/L). These strains are from the species *Lactobacillus crispatus* and *Lactobacillus gasseri*, respectivley, and were found in the natural vaginal enviroment of females. The DSM 35171 produces only 0.83 g/L total lactate, but shows strong properties in the pH lowering effect showing that this strain produces other organic acids besides lactate to reduce the pH (s. example 2).

### Example 2: Determination of the rate of acidification (pH lowering effect) by Pediococcus pentosaceus DSM 35171, Lactobacillus crispatus DSM 35177, Lactiplantibacillus gasseri DSM 35178, Lactiplantibacillus plantarum DSM 35176

The strains were isolated from female vaginal swabs described in Example 1. DSM 35171, DSM 35177, DSM 35178 and DSM 35176 were cultivated in 10 ml MRS broth (see Example 1) in a 15 ml tube for 24 h at 37 °C under anaerobic conditions (without agitation). Bacterial cells were harvested by centrifugation (7500 rpm, 10 min), the pellet was washed with saline solution (0.9 % NaCl) and resuspended in MRS broth to obtain a 10⁹ cells/mL bacterial suspension (OD₆₂₀ of 0.25). The cultures were then incubated at 37 °C under anaerobic conditions. The rate of acidification was evaluated measuring in duplicate the pH after 2, 4, 6, 8, 16, and 24 h. The initial pH was measured (pH 6.3) and the rate of acidification was determined by calculating the delta pH over time.

Strains of interest, which were isolated from female vagina, were compared to strains which were already available in products of the female health market.

**Table 2: pH decrease in a suspension with 10⁹ cells/mL bacterial cells of tested strains. Mean values of two independent cultivations are depicted as total pH decrease during 24 hours. Strains were cultivated in MRS medium. The initial pH of all samples was adjusted to pH 6.2.**

| **Strain** | **Species** | **pH decrease = pH lowering effect** |
|---|---|---|
| | | **(Δ0-24h)** |
| **DSM 35176** | *Lactiplantibacillus plantarum (+)* | 3.23 |
| **DSM 35177** | *Lactobacillus crispatus (+)* | 3.11 |
| **DSM 35171** | *Pediococcus pentosaceus (+)* | 3.1 |
| **DSM 35178** | *Lactobacillus gasseri (+)* | 2.91 |
| **Evonik strain 5** | *Lactobacillus crispatus (-)* | 1.45 |
| **Evonik strain 6** | *Lactobacillus gasseri (-)* | 2.37 |
| **Evonik strain 7** | *Lactiplantibacillus plantarum (-)* | 2.39 |
| **Evonik strain 8** | *Pediococcus pentosaceus (-)* | 2.79 |
| **Competitor strain 1** | *Lactobacillus gasseri* | 2.55 |
| **Competitor strain 2** | *Lactobacillus crispatus* | 1.62 |
| **Competitor strain 3** | *Lactobacillus jensenii* | 2.00 |
| **Competitor strain 4** | *Lactobacillus crispatus* | 2.45 |

As highlighted in Table 1 DSM 35176, DSM 35177, DSM 35171 and DSM 35178 have the ability to change the pH during a 24h-cultivation by 3.23-2.91 units to a more acidic condition. The change in pH value is exceptionally good with the selected strains, and no better performers could be found. Strains of the same species and strains available also in in commercial products were tested for comparison. None of these strains had the ability to lower the pH as much as the strains of the invention.

### Example 3: Survival of Pediococcus pentosaceus DSM 35171 and Lactiplantibacillus plantarum DSM 35176 in simulated gastric and intestinal fluids under in vitro conditions

The lactic acid bacteria ability to survive during the gastrointestinal (GI) transit was done *in vitro* using simulated gastric fluid (SGF) and simulated intestinal fluid (SIF), as described by Fernandez, Boris, and Barbes (2003), with some modifications. In detail, SGF was prepared with NaCl (125 mM/L), KCI (7mM/L), NaHCO3 (45 mM/L), pepsin (3 g/L) (Sigma-Aldrich CO., St. Louis, MO, USA), and adjusted to pH 2 (with HCl). As in Example 1 explained, stationary-phase-grown cells (24 h) were harvested (7,500 rpm, 10 min), washed with saline solution standardized at 9 Log CFU/mL and resuspended in SGF. The bacterial suspensions were incubated under anaerobic conditions and stirring (150 rpm) at 37 °C to simulate peristalsis of the gastric transit. Samples for total viable counts were taken after 3 h of incubation. Subsequently, grown bacterial suspensions from the prior incubation in SGF were collected by centrifugation (7,500 rpm, 10 min) and resuspended to the original volume in SIF, which contained pancreatin (0.1%, w/v) and bile salts (0.15%, w/v, Sigma-Aldrich Co.) adjusted to pH 8.0 (with NaOH). The suspensions were incubated as in SGF and samples for total viable counts were taken after 3 h of the intestinal transit. Appropriate dilutions of the samples were made and plated on MRS-agar plates (Roth, X925.1+15 g/L agar-agar). Plates were incubated anaerobically at 37°C and colonies were counted after 48 h. The Delta T0-T2 ≤ 2 log cycles were evaluated as high resistant to simulated gastric and intestinal fluids.

**Table 3: The survival of stationary grown cells was tested in simulated gastric and intestinal fluids to simulate the GIT transition. An initial log 9 CFU/ml was adjusted and survival was tested after three hours of incubation in SIF (T1) as well as after subsequent further three hours of incubation in SGF (T2) by a plating approach. A reduction of ≤ 2 log cycles was considered as high resistance.**

| **Strain** | **T0 log CFU/ml** | **T1 log CFU/ml** | **T2 log CFU/ml** | **T0-T2** |
|---|---|---|---|---|
| **DSM 35171** | 9.0 (STD 0.53) | 8.92 (STD 0.21) | 7,01 (0.1) | 1.99 |
| **DSM 35176** | 9.0 (STD 0.84) | 8.60 (STD 0.09) | 7.11 (STD 0.07) | 1.89 |

### Example 4: Adhesion assay of Pediococcus pentosaceus DSM 35171 and Lacticaseibacillus rhamnosus DSM 35175 at cells of vaginal tissue

VK2E6/E7 (CRL-2616 ^{™}, ATCC^{®}) cells were cultivated as disclosed in (Fichorova RN, 1997), applying some modifications that will be listed next. Cells were cultivated in Keratinocyte-Serum Free Medium (Gibco, 10725-018) added with 0.1ng/ml Epidermal growth factor (Gibco, 10450-013), 0.05mg/ml bovine pituitary extract (Gibco, 13028- 014), CaCl2 (finale concentration 0.4 mM, Roth, CN93.4) and Penicillin-Streptomycin (Sigma, P4333-100ml) in 75 cm³ cell-culture flasks (Roth, Greiner Bio-One) at 37°C in an incubator with 5% CO₂ without shaking. The medium was changed every two days. The cells were passaged when reaching 70% confluency. The cells were harvested and seeded in 48-wells plates (Faust Lab, TPP92048) at 6E+04 cells/wells. After reaching confluency, the cell-monolayers were further cultivated for 7 days to allow differentiation. The medium was changed every two days. Each well contained about 2E+05 cells.

DSM 35171 and DSM 35175 were grown in De Man-Rogosa-Sharpe agar (MRS; Roth, X925.1) added with 0.5g/L Cysteine-HCL (Carl Roth, 1694.3). MRS contains 10g/L peptone, 4 g/L yeast extract, 8 g/L meat extract, 20 g/L glucose, 2 g/L dipotassium phosphate, 5 g/L sodium acetate, 2 g/L ammonium acetate, 0.2 g/L magnesium sulfate, 0.05 g/L manganese sulfate, 1 g/L polysorbate 80 and the pH was adjusted to pH 6.2 (+/- 0.2). For the cultivation on plate MRS with 1.5% agar-agar was used. Bacterial strains were cultivated on MRS-agar plates for 24 hours at 37°C in an anaerobic box added with an AnaeroGenTM bag (Thermo Fisher, AN0035A). For liquid cultivation, 15 ml cultivation tubes were filled with 10 ml MRS broth with cysteine and bacterial material from the agar plate was used as inoculum. Liquid cultivations were incubated at 37°C for 24 hours without shaking anaerobically.

At experimental day, the VK2E6/E7 cells were washed with pre-warmed 1x Phosphate-Buffered-Saline (PBS, ROTI^{®}Cell, 10x) followed by addition of 250µl growth medium, but without addition of antibiotics. The cells were then incubated at 37°C and 5% CO2 for 2 hours in an incubator without shaking, as described above.

Bacterial overnight cultures were washed and resuspended in PBS. The bacterial concentration was determined microscopically and adjusted to 1x10⁸ cells/ml. The bacteria were diluted 1/10 in culture medium ("solution A", cell-culture medium (Keratinocyte-Serum Free Medium(Gibco, 10725-018) added with 0.1ng/ml Epidermal growth factor (Gibco, 10450-013), 0.05mg/ml bovine pituitary extract (Gibco, 13028- 014), CaCl2 (final concentration 0.4 mM, Roth, CN93.4))) and 250µl (2.5x10⁶ bacteria) were added onto the VK2E6/E7 cells in triplicate and incubated at 37°C for 90 minutes in an incubator with 5% CO2. As reference ("solution B"), 250µl of solution A were added to 250µl medium without antibiotics (without VK2E6/E7 cells) and incubated at 37°C and 5% CO2 for 90 minutes.

As control, solution A was directly diluted, and several dilutions put on appropriate agar to determine CFU. After the 90 minutes of incubation, the solution B was also diluted, and several dilutions putted on appropriate agar to determine CFU.

The viability of the bacteria at the end of the incubation was determined as follows:$\left(CFU in solution B / CFU in colution A\right) \times 100$

After incubation, the cells were washed three times with pre-warmed PBS to remove unbound bacteria. The cells were harvested by incubation with trypsin and 0.1% triton-X 100 at 37°C. The released cells and the bound bacteria ("Solution C") were appropriately diluted, and several dilutions putted on appropriate agar to determine CFU.

The percentage of bacteria bound to the cells was determined as followed:$\left(CFU in solution C / CFU in solution B\right) X 100$

Each strain was tested for binding on VK2E6/E7 cells in triplicate in at least two independent experiments. Binding capacity is given in percentage [%] from mean values.

Each strain included in this study was tested in at least two independent experiments. All strains survived well the 90 minutes incubation in culture medium at 37°C and 5% CO2. Furthermore, the strains did not significantly overgrowth in the medium along the 90 minutes incubation. Consequently, no adjustment was needed and a survival rate between 80% and 120% was set as range to validate the results.

The ATCC53103 strain was seen as internal benchmark which shows a binding property of 8.33% +/-2.5. Some of the tested strains presented similar or better binding property as the control strain (Table 1). Among them, one strain, the DSM 35175 presented a binding at least 25% (factor 1.25) above the binding observed with the control strain. With a binding capacity of 14.90% and a factor of 1.79 the strain of the species *Lacticaseibacillus rhamnosus* is outcompeting the ATCC53103 with better binding properties. Only one strain, the DSM 35171, is even better. The *Pediococcus pentosaceus,* isolated from the female vagina shows a relative increase factor of 3 and 25% binding capacity. None of the other tested strains from the Evonik strain collection or comparative strains show that high binding capacity.

**Table 4: Binding of DSM 35171, DSM 35175, ATCC53103 and other tested strains to the VK2E6/E7. For each strain the mean value and standard deviation of binding is presented in percentage (%). The relative increase in binding is compared to the ATCC53103 strain and given as factor increase.**

| **Strain** | **Species** | **Binding (%)** | **STD (%)** | **Factor increase relative to control** |
|---|---|---|---|---|
| **DSM 35171*** | *Pediococcus pentosaceus* | 24.96 | 2.26 | 3.00 |
| **DSM 35175**** | *Lacticaseibacillus rhamnosus* | 14.90 | 0.28 | 1.79 |
| **ATCC53103 (C)** | *Lacticaseibacillus rhamnosus* | 8.33 | 2.49 | control |
| **Competitor strain 1** | *Lacticaseibacillus rhamnosus* | 0.80 | 0.16 | 0.10 |
| **Evonik strain 3**** | *Pediococcus pentosaceus* | 8.98 | 1.72 | 1.08 |
| **Evonik strain 4**** | *Lacticaseibacillus rhamnosus* | 8.87 | 2.52 | 1.07 |
| **Evonik strain 5**** | *Lacticaseibacillus rhamnosus* | 2.15 | 1.81 | 0.26 |

| | | | | |
|---|---|---|---|---|
| * isolated from the female vagina ** isolated from food sources (C) *Lactobacillus rhamnosus* GG (LGG) ^{®}, ATCC53103 | | | | |

As can be seen, the strain ATCC53103, which was seen as internal benchmark, showed a binding capacity of 8.33% +/-2.5. Some of the tested strains showed a similar or even better binding property than this reference strain. Among them, the strain *Lacticaseibacillus rhamnosus* DSM 35175 exhibited a binding at least 25% (factor 1.25) above the binding observed with the reference strain. With a binding capacity of 14.90% and a factor of 1.79, the strain DSM 35175 is outperforming the strain ATCC 53103 with better binding properties. Only one strain, DSM 35171, exhibits even better binding capacity. The *Pediococcus pentosaceus,* isolated from the female vagina shows a relative increase factor of 3 and 25% binding capacity. None of the other tested strains from the Evonik strain collection or comparative strains did show such a high binding capacity.

### Example 5: Determining the inhibitory activity against Gardnerella vaginalis with cell-free supernatants (pH 4 or pH 7) of Lactiplantibacillus gasseri DSM 35178, Lactiplantibacillus plantarum DSM 35176 and Limosilactobacillus fermentum DSM 35179

Strains were isolated as described above. For the inhibition assay, cell-free supernatants were generated. Strains were cultivated in 10mL MRS broth for 24 h at 37 °C under anaerobic conditions. The anaerobically grown cells were cultivated in a 15 ml tube without agitation. For the determination of pathogen control, anaerobically grown cells were adjusted to 10⁹ cells/mL (OD620 of 0.25). After the adjustments the cell-free supernatants (CFSs) were created by centrifugation (7500 rpm, 10 min) and were fractionated in two 1 ml aliquots. One aliquot was neutralized at pH 7.0 (n-CFSs) with NaOH and sterilized by using a 0.22 µm filter. The other aliquot was preserved at the original acidic pH of ca. 4.0 (o-CFSs) and was sterilized by using a 0.22 µm filter. All aliquots of CFSs were stored at -20°C no more than a week.

*G. vaginalis* was cultured on peptone-starch dextrose blood medium and incubated under anaerobic conditions at 37 °C for 3 days. The growth inhibition against this pathogen was assessed using filtered CFSs, previously collected and stored. Briefly, overnight cultures of the pathogen were washed twice with saline solution and resuspended in ATCC medium 1685 to a final OD620 (λ=620 nm) of 0.25 for G. *vaginalis.* The 96-well plates were set using one volume of CFSs and three volumes of each pathogen. The analysis was done in duplicate using CFSs previously neutralized (pH 7.0) and the acidic CFSs (pH ca. 4.0). Control wells were prepared using MRS medium at pH 4.0, and MRS at pH 7.0, also using the respective broth medium of the pathogen, and run in duplicate. Growth kinetics of the pathogen was recorded for 46 h at 37 °C measuring the absorbance (at a wavelength of 620 nm for *G*. *vaginalis*) every 15 min with the Infinite^{®} M Nano+Spectrophotometer (TECAN, Austria). The recorded highest absorbance within 46 h was used as maximal absorbance value, which counts as the highest optical density.

Growth kinetics of *G*. *vaginalis* were assessed in the presence of cell-free supernatants (CFSs) from a broad range of lactic acid bacteria strains and compared against the control condition (without CFSs). The inhibitory activity was tested using CFSs at the original pH of 4.0 (o-CFSs) or neutralized CFSs at pH 7 (n-CFSs), to exclude pH-dependent effects. For *G*. *vaginalis* the parameter absorbance (A) and growth rate (µ) were monitored. *G. vaginalis* grown at pH 7.0 as a control condition reached a maximum absorbance (A) of 1.19 ± 0.06 and growth rate (µ) of 0.2 ± 0.01 h⁻¹. At pH 4.0, lower A (0.47 ± 0.071) and smaller µ (0.08 ± 0.01 h⁻¹) were reached.

The n-CFSs and o-CFSs of DSM 35176, DSM 35178 and DSM 35179 show the ability to at least reduce the growth (absorbance and growth rate) or inhibit the growth of G. *vaginalis* completely (Table 2). DSM 35179, which already has the potential to reduce the growth of *Candida albicans,* shows a complete growth inhibition of *Gardnerella vaginalis,* when n-CFS or O-CFS are used. Besides that, n-CFS of DSM *35176* (*Lactiplantibacillus plantarum*) and o-CFS of DSM 35178 (*Lactobacillus gassen*) also show complete growth inhibition of *G*. *vaginalis.* The o-CFS of DSM 35176 reduces the growth of G. *vaginalis* to 0.14 A and causes a smaller growth rate. This is also the case for the n-CFS of DSM 35178 (0.18 A and 0.01 µ) (Table 2). All three strains, isolated from the female vagina, can be used as beneficial BV-treatments.

**Table 5: Growth inhibition of Gardnerella vaginalis from cell-free supernatants kept at original acidic pH (pH 4; o-CFS) or adjusted to neutral pH (pH 7; n-CFS) for tested strains DSM 35176, DSM 35178, DSM 35179 and DSM 35180 in comparison to strains selected for a comparative analysis and in comparison to the negative control of growth of Gardnerella vaginalis without addition of lactic acid bacteria (CTR). If no growth of G. vaginalis could be monitored, "complete inhibition" is depicted.**

| | | pH 7 (n-CFS) | pH 4 (o-CFS) |
|---|---|---|---|
| ID | Species | A | A |
| Control (CRT) | *G. vaginalis* | 1.19 ± 0.06 | 0.47 ± 0.071 |
| DSM 35176 | *Lactiplantibacillus plantarum* | complete inhibition | 0.18 |
| DSM 35179 | *Limosilactobacillus fermentum* | complete inhibition | complete inhibition |
| DSM 35178 | *Lactobacillus gasseri* | 0.18 | complete inhibition |
| Competitor strain 1 | *Lactiplantibacillus plantarum* | 0.78 | 0.16 |
| Competitor strain 2 | *Lactobacillus gasseri* | 0.71 | complete inhibition |
| Evonik strain 4 | *Lactiplantibacillus plantarum* | 1.01 | ND |
| Evonik strain 5 | *Limosilactobacillus fermentum* | 0.84 | 0.58 |
| Evonik strain 6 | *Lactobacillus gasseri* | 0.79 | 0.14 |

### Example 6: Determining the inhibitory activity against Candida albicans and Gardnerella vaginalis with cell-free supernatants (pH 4 or pH 7) of Lactiplantibacillus plantarum DSM 35176, Lactobacillus gasseri DSM 35178, Limosilactobacillus fermentum DSM 35179 and Lactiplantibacillus plantarum DSM 35180

Strains were isolated as described above. For the inhibition assay, cell-free supernantants were generated. Strains were cultivated in 10mL MRS broth for 24 h at 37 °C under anaerobic conditions. The anaerobically grown cells were cultivated in a 15 ml tube without agitation. For the determination of pathogen control, anaerobically grown cells were adjusted to 109 cells/mL (OD620 of 0.25). After the adjustments the cell-free supernatants (CFSs) were created by centrifugation (7500 rpm, 10 min) and were fractionated in two 1 ml aliquots. One aliquot was neutralized at pH 7.0 (n-CFSs) with NaOH and sterilized by using a 0.22 µm filter. The other aliquot was preserved at the original acidic pH of ca. 4.0 (o-CFSs) and was sterilized by using a 0.22 µm filter. All aliquots of CFSs were stored at -20°C no more than a week.

*C. albicans* was grown aerobically in Sabouraud medium (Sharlau, Spain) at 35 °C. The growth inhibition against *C*. *albicans* was assessed using filtered CFSs, previously collected and stored. Briefly, overnight cultures of the pathogen were washed twice with saline solution and resuspended in Sabouraud medium to a final OD600 of 0.1. 96-well plates were set using one volume of CFSs and three volumes of the pathogen. The analysis was done in duplicate using CFSs previously neutralized (pH 7.0) and the acidic CFSs (pH ca. 4.0). Control wells were prepared using MRS medium at pH 4.0, and MRS at pH 7.0, also using the respective broth medium of the pathogen, and run in duplicate. Growth kinetics of the pathogen were recorded for 46 h at 37 °C measuring the absorbance (at a wavelength of 600 nm) every 15 min with the Infinite^{®} M Nano+Spectrophotometer (TECAN, Austria). The recorded highest absorbance within 46 h was used as maximal absorbance value, which counts as the highest optical density.

Growth kinetics of *C*. *albicans* were assessed in the presence of cell-free supernatants (CFSs) from a broad range of lactic acid bacteria strains and compared against the control condition (without CFSs). The inhibitory activity was tested using CFSs at the original pH of appr. 4.0 (o-CFSs) or neutralized CFSs at pH 7 (n-CFSs), to exclude pH-dependent effects. *C. albicans* grown at pH 7.0 as a control condition reached a maximum absorbance (A) of 1.24 ± 0.02, with the lag phase (A) 3.85 ± 0.17 h, and growth rate (µ) 0.20 ±0.01 h⁻¹. At pH of 4.0, lower A (1.12 ± 0.02), longer (4.19 ± 0.44 h) but similar µ (0.19 ±0.01 h⁻¹) were reached.

If *C*. *albicans* was cultured with n-CFSs or o-CFSs of DSM 35179 or DSM 35180, then lower absorbance and reduced growth rates were observed in comparison to the control condition. This shows that both lactic acid bacteria strains, especially the produced metabolites, are able to inhibit the growth of *C*. *ablicans* (Table 1). The n-CFS of DSM 35179 of the species *Limosilactibacillus fermentum* causes in addition a reduced absorbance (-0.17 A) and a lower growth rate (-0.03 h⁻¹) of *Candida albicans.* From the same strain the o-CFS causes a higher reduction of the total absorbance (-0.22 A) and also a reduced growth rate (-0.02 h⁻¹). Besides DSM 35179, also the strain DSM 35180 (*Lactiplantibacillus plantarum*), lowers the final absorbance of *Candida albicans* with its n-CFS (-0.22 A; -0.07 h⁻¹). Compared to strains of the same species or strains which are already in other products, DSM 35179 and DSM 35180 stand out with their performance (see Table 6).

**Table 6: Growth inhibition of Candida albicans from cell-free supernatants kept at original acidic pH (pH 4; o-CFS) or adjusted to neutral pH (pH 7; n-CFS) for tested strains DSM 35179 and DSM 35180 in comparison to strains selected for a comparative analysis and in comparison to the negative control of growth of C. albicans without addition of lactic acid bacteria (CTR).**

| | | pH 7 (n-CFS) | pH 4 (o-CFS) |
|---|---|---|---|
| ID | Species | A | A |
| Control (CTR) | *C. albicans* | 1.24 | 1.12 |
| DSM 35179 | *Limosilactobacillus fermentum* | 1.07 | 0.90 |
| DSM 35180 | *Lactiplantibacillus plantarum* | 1.02 | 0.93 |
| DSM 35176 | *Lactiplantibacillus plantarum* | 1.16 | 1.12 |
| DSM 35178 | *Lactobacillus gasseri* | 1.16 | 1.10 |
| Evonik strain 5 | *Limosilactobacillus fermentum* | 1.28 | 0.98 |
| Evonik strain 6 | *Lactiplantibacillus plantarum* | 1.17 | 1.19 |

### Literature

Bagnall, Paulette, and Denise Rizzolo. 2017. "Bacterial Vaginosis: A Practical Review." Journal of the American Academy of PAs 30 (12).
De Angelis, Maria, Sonya Siragusa, Mariagrazia Berloco, Leonardo Caputo, Luca Settanni, Giuditta Alfonsi, Marica Amerio, Augusto Grandi, Adriano Ragni, and Marco Gobbetti. 2006. "Selection of Potential Probiotic Lactobacilli from Pig Feces to Be Used as Additives in Pelleted Feeding." Research in Microbiology 157 (8): 792-801. https://doi.org/10.1016/j.resmic.2006.05.003.
Fernandez, M.F., S. Boris, and C. Barbes. 2003. "Probiotic Properties of Human Lactobacilli Strains to Be Used in the Gastrointestinal Tract." Journal of Applied Microbiology 94 (3): 449-55. https://doi.org/10.1046/j.1365-2672.2003.01850.x.
Javed, Ayesha, Fahed Parvaiz, and Sobia Manzoor. 2019. "Bacterial Vaginosis: An Insight into the Prevalence, Alternative Treatments Regimen and It's Associated Resistance Patterns." Microbial Pathogenesis 127: 21-30. https://doi.org/10.1016/j.micpath.2018.11.046.
Jones, Ashley. 2019. "Bacterial Vaginosis: A Review of Treatment, Recurrence, and Disparities." The Journal for Nurse Practitioners 15 (6): 420-23. https://doi.org/10.1016/j.nurpra.2019.03.010.
Kahm, Matthias, Guido Hasenbrink, and Jost Ludwig. 2010. "Grofit: Fitting Biological Growth Curves with R." Journal of Statistical Software 33 (7): 1. https://doi.org/10.18637/jss.v033.i07.
Moosa, Yumna, Douglas Kwon, Tulio de Oliveira, and Emily B. Wong. 2020. "Determinants of Vaginal Microbiota Composition." Frontiers in Cellular and Infection Microbiology 10 (September): 1-9. https://doi.org/10.3389/fcimb.2020.00467.
Ravel, J., P. Gajer, Z. Abdo, G. M. Schneider, S. S. K. Koenig, S. L. McCulle, S. Karlebach, et al. 2011. "Vaginal Microbiome of Reproductive-Age Women." Proceedings of the National Academy of Sciences 108 (Supplement_1): 4680-87. https://doi.org/10.1073/pnas.1002611107.
Rodriguez-Cerdeira, Carmen, Miguel Carnero Gregorio, Alberto Molares-Vila, Adriana López-Barcenas, Gabriella Fabbrocini, Brunilda Bardhi, Ardiana Sinani, Elena Sánchez-Blanco, Roberto Arenas-Guzmán, and Rigoberto Hernandez-Castro. 2019. "Biofilms and Vulvovaginal Candidiasis." Colloids and Surfaces B: Biointerfaces 174: 110-25. https://doi.org/10.1016/j.colsurfb.2018.11.011.
Shenoy, Adele, and Alice Gottlieb. 2019. "Probiotics for Oral and Vulvovaginal Candidiasis: A Review." Dermatologic Therapy 32 (4): e12970. https://doi.org/10.1111/dth.12970.
Tachedjian, Gilda, Muriel Aldunate, Catronia S. Bradshaw, and Richard A. Cone. 2017. "The Role of Lactic Acid Production by Probiotic Lactobacillus Species in Vaginal Health." Research in Microbiology. https://doi.org/10.1016/j.resmic.2017.04.001.
Witkin, S. S., and I. M. Linhares. 2017. "Why Do Lactobacilli Dominate the Human Vaginal Microbiota?" BJOG: An International Journal of Obstetrics and Gynaecology 124 (4): 606-11. https://doi.org/10.1111/1471-0528.14390.

## Claims

1. Microorganism with the ability to strongly reduce the pH of the surrounding medium and preparations of such a microorganism selected from the following group:
a) *Pediococcus pentosaceus* DSM 35171;
b) *Lactobacillus crispatus* DSM 35177;
c) *Lactiplantibacillus gasseri* DSM 35178;
d) *Lactiplantibacillus plantarum* DSM 35176;
e) a mutant of a strain according to (a) to (d) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
f) a preparation of a microorganism according to (a) to (e), wherein the preparation is preferably an optionally concentrated or dried fermentation broth, supernatant of a fermentation broth, ferment, cell lysate or cell extract of a microorganism according to (a) to (e), wherein the preparation may be cell-free or may contain living or dead cells or cell debris of such a microorganism or any combination thereof.

2. Microorganism or microbial preparation according to claim 1, wherein the microorganism is able to produce high amounts of lactic acid, in particular of D-lactic acid.

3. Microorganism or microbial preparation according to claim 1 or 2, wherein the microorganism possesses at least one, preferably at least two or three, of the following further characteristics:
- Ability to grow in presence of 0.15 wt.-% of bile;
- Ability to survive exposure to a pH of 2 for at least 2 hours, preferably with a survival rate of at least 5 %, more preferably of at least 10 %;
- Ability to survive the exposure to 3 g/L pepsin at 37°C for at least 2 hours, preferably with a survival rate of at least 5 %, more preferably of at least 10 %;
- Ability to bind to cells of the vaginal tissue, in particular to VK2E6/E7 cells ;
- Ability to inhibit the growth of pathogenic bacteria, in particular *Gardnerella vaginalis.*

4. Composition comprising a microorganism or a microbial preparation according to any of claims 1 to 3, wherein the composition preferably contains at least one, more preferably at least two or three, further substances selected from estriol, mannose, lactic acid, glycogen, cinnamon, carriers, further probiotics, prebiotics, in particular inulin and/or xylo-oligosaccharid, enzymes, vitamins, in particular vitamin A, vitamin B1 , vitamin B2, vitamin B6, vitamin B12, biotin, niacin, folic acid, pantothenate, vitamin C and/or vitamin D, minerals, in particular zinc and/or selenium, amino acids, peptides, plant extracts, in particular cranberry extract, myrrh extract, chamomile extract, sour cherry extract and/or oat extract, yeast extracts, and algal extracts.

5. Composition according to claim 4, wherein the composition is a composition for oral administration, preferably selected from food and feed compositions, food and feed supplements, food and feed additives, dietary supplements, nutraceuticals and functional foods and preferably contains at least one further food or feed additive, in particular selected from proteins, carbohydrates, fats, feed concentrates, silage, mashfeed, immune modulators, milk replacers, acid-based products and/or medicines, in particular antibiotics.

6. Composition according to claim 4, wherein the composition is a cosmetical composition, preferably for topical application, and preferably contains at least one further additive selected from surfactants, emulsifiers, phospholipids, sphingoid bases, free fatty acids, protein hydrolysates, polymers, light stabilizers, anti-perspirants, deodorants, cosmetic oils, emollients, antioxidants, preservatives, thickeners, viscosity regulators, stabilizers, hydrotropes, solids, fillers, film formers, conditioners, insect repellents, self-tanning agents, odor absorbers, solvents, perfumes and dyes and wherein the composition is preferably a suppository, a cream or a gel.

7. Pharmaceutical composition, in particular for oral or topical administration, containing a pharmaceutically acceptable carrier and at least one microorganism or microbial preparation according to any of claims 1 to 3.

8. Composition according to any of claims 4 to 7, wherein the composition comprises living cells of such microorganisms, preferably in an amount of from 1x10² to 1x10¹² CFU, more preferably in an amount of 1x10³ to 1x10¹⁰ CFU, above all in an amount of 1x10⁴ to 1x10⁹ CFU.

9. Composition according to any of claims 4 to 8, wherein the composition contains at least one further microorganism or microbial preparation, wherein the microorganism or microbial preparation have the ability to inhibit the growth of *Candida albicans* and/or *Gardnerella vaginalis* and wherein the microorganism is preferably a lactic acid bacterium and wherein the microorganism or microbial preparation is very preferably selected from the following group:
a) *Limosilactobacillus fermentum* DSM 35179;
b) *Lactiplantibacillus plantarum* DSM 35180;
c) a mutant of a strain according to (a) or (b) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
d) a preparation of a microorganism according to (a) to (c), wherein the preparation is preferably an optionally concentrated or dried fermentation broth, supernatant of a fermentation broth, ferment, cell lysate or cell extract of a microorganism according to (a) to (c), wherein the preparation may be cell-free or may contain living or dead cells or cell debris of such a microorganism or any combination thereof.

10. Composition according to any of claims 4 to 9, wherein the composition contains at least one further microorganism or microbial preparation, wherein the microorganism or microbial preparation has the ability to bind to vaginal tissue, in particular to to VK2E6/E7 cells, and wherein the microorganism or microbial preparation is preferably selected from the following group:
a) *Lacticaseibacillus rhamnosus* DSM 35175;
b) a mutant of a strain according to (a) with a sequence identity to the genomic DNA of such a strain of at least 95 %, preferably of at least 97, 98 or 99 %, more preferably of at least 99.5, 99.8 or 99.9 %, in particular of at least 99.95, 99.98 or 99.99 %;
c) a preparation of a microorganism according to (a) or (b), wherein the preparation is preferably an optionally concentrated or dried fermentation broth, supernatant of a fermentation broth, ferment, cell lysate or cell extract of a microorganism according to (a) or (b), wherein the preparation may be cell-free or may contain living or dead cells or cell debris of such a microorganism or any combination thereof.

11. Composition according to any of claims 4 to 10, wherein the composition is a coated composition and/or contained in a capsule, wherein the coated composition comprises preferably an enteric coating, in particular comprising methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, shellac, cellulose acetate trimellitate, sodium alginate or zein or combinations thereof and wherein the capsule is preferably a bile-resistant capsule.

12. Product containing a microorganism or microbial preparation according to any of claims 1 to 3 or a composition according to any of claims 4 to 11, wherein the product is preferably a personal care article or a medical device, wherein the personal care article is preferably a disposable absorbent article, in particular selected from sanitary napkins, panty liners, tampons, diapers, incontinent pads, breast pads, perspiration pads, human or animal waste management devices and interlabial pads.

13. Microorganism or microbial preparation according to any of claims 1 to 3 or composition according to any of claims 4 to 11 or product according to claim 12 for use in the treatment, prevention or mitigation of the course of a disease or condition related to pathogens of the vaginal tissue, wherein the pathogens are preferably selected from *E*. *coli* UPEC (uropathogenic *E. coli*), *Atopobium vaginae, Candida albicans* and *Gardnerella vaginalis* and wherein the disease or condition is preferably selected from diseases and conditions associated with such pathogenic bacteria, in particular selected from infections of the urinary tract, bacterial vaginosis, vulvovaginal candidiasis, endometriosis, intestinal candidiasis, irritable bowel syndrome (IBS), preterm labor, miscarriage, vaginal itchiness and burning, vaginal and intestinal infections, in particular vaginal and intestinal infections by bacteria or yeast of Candida species, mental, mood and/or sleep disorders or diseases, in particular pain, anxiety, stress and/or loss of motor control.

14. Use of a microorganism or microbial preparation according to any of claims 1 to 3 or of a composition according to any of claims 4 to 11 in the manufacture of a pharmaceutical composition for treating or preventing or mitigating a disease or condition which is related to pathogenic bacteria of the vaginal tissue, wherein the pathogenic bacteria are preferably selected from *E*. *coli* UPEC (uropathogenic *E*. *coli*), *Atopobium vaginae, Candida albicans* and *Gardnerella vaginalis* and wherein the disease or condition is preferably selected from diseases and conditions associated with such pathogenic bacteria, in particular selected from infections of the urinary tract, bacterial vaginosis, vulvovaginal candidiasis, endometriosis, intestinal candidiasis, irritable bowel syndrome (IBS), preterm labor, miscarriage, vaginal itchiness and burning, vaginal and intestinal infections, in particular vaginal and intestinal infections by bacteria or yeast of Candida species, mental, mood and/or sleep disorders or diseases, in particular pain, anxiety, stress and/or loss of motor control.

15. Non-medical use of a microorganism or microbial preparation according to any of claims 1 to 3 or of a composition according to any of claims 4 to 11 or of a product according to claim 12 for alleviating or preventing mental, mood and/or sleep disorders or disbalances, in particular anxiety and/or stress, and/or for supporting vaginal health, in particular for supporting the natural barrier function of the vaginal tissue, for protecting the vaginal tissue against specific pathogens, for promoting a healthy microbiome, for enhancing natural defenses, for preventing itching, burning and/or bad odor of the female genital area.

16. Method of producing lactic acid, in particular D-lactic acid, wherein a microorganism according to any of claims 1 to 3 is employed, wherein the microorganism is cultivated in a suitable fermentation medium and after the fermentation is finished the supernatant is preferably separated from the cell mass and optionally dried.
